# EUROPEAN PATENT APPLICATION

(11) **EP 2 491 875 A2**
(43) Date of publication of application: **29.08.2012**
(21) Application number: 12156834.9
(22) Date of filing: 24.02.2012
(51) Int. Cl.: A61B 17/3203

(54) **Fluid ejection device**

(30) Priority: 28.02.2011 JP 2011041140
(71) Applicant: Seiko Epson Corporation, Shinjuku-ku Tokyo (JP)
(72) Inventor: Kojima, Hideki, Nagano, 392-8502 (JP); Seto, Takeshi, Nagano, 392-8502 (JP); Hirabayashi, Atsuya, Nagano, 392-8502 (JP)
(74) Representative: HOFFMANN EITLE

(57) **Abstract**

A surgical instrument (1) includes: a fluid chamber (80) to which fluid is supplied; a pulsation generator (20) which produces pulsed flow by pressurizing the fluid supplied to the fluid chamber (80); an outlet channel (82) which communicates with the fluid chamber (80) through which the pulsed flow is transmitted; a fluid ejection opening (93) which communicates with the outlet channel (82) through which pulse flow is ejected toward an ejection target; a suction opening (96) provided in the vicinity of the fluid ejection opening (93); and a fluid suctioning unit which suctions the fluid through the suction opening (96), wherein the opening area at an outlet opening end (93b) of the fluid ejection opening (93) on the side near the ejection target is larger than the opening area at an inlet opening end (93a) of the fluid ejection opening (93) on the side near the outlet channel (82).

## Description

### BACKGROUND

### 1. Technical Field

The present invention relates to a surgical instrument which excises or incises a living body by using ejected fluid.

### 2. Related Art

Currently, such a surgical instrument has been proposed which converts fluid into pulsed flow by rapid change of the volume of a fluid chamber. This rapid volume change is produced by a volume changing unit. Thus, the fluid is ejected at high speed in pulses through a nozzle for incision or excision of living tissue (for example, see JP-A-2008-82202).

According to this type of surgical instrument, fluid continues to be supplied to the fluid chamber even while the volume changing unit stops operation. In this case, the fluid flows out from a fluid ejection opening during stopping of the pulsed flow ejection. To avoid this problem, an improved type of surgical instrument has been proposed which includes a micro-valve disposed in an inlet channel extending from a fluid supply unit to the fluid chamber to prevent fluid flow out of the fluid ejection opening during stopping of the volume changing unit (see JP-A-2009-285116).

According to the technology disclosed in JP-A-2009-285116, however, the additional unit of the micro-valve complicates the structure of the device.

### SUMMARY

An advantage of some aspects of the invention is to provide a technology which solves at least a part of the aforementioned problems by reducing flow out of fluid during stop of pulsed flow ejection without requiring complicated structure.

### Application Example 1

This application example of the invention is directed to a surgical instrument including: a fluid chamber to which fluid is supplied; a pulsation generator which produces pulsed flow by pressurizing the fluid supplied to the fluid chamber; an outlet channel which communicates with the fluid chamber through which the pulsed flow is transmitted; a fluid ejection opening which communicates with the outlet channel through which pulse flow is ejected toward an ejection target; a suction opening provided in the vicinity of the fluid ejection opening; and a fluid suctioning unit which suctions the fluid through the suction opening. The opening area at an outlet opening end of the fluid ejection opening on the side near the ejection target is larger than the opening area at an inlet opening end of the fluid ejection opening on the side near the outlet channel.

According to the surgical instrument of Application Example 1, the fluid supplied to the fluid chamber is pressurized to generate pulsed flow which achieves ejection of the fluid in pulses through the fluid ejection opening. While the pressurization and depressurization of the fluid supplied to the fluid chamber is being stopped (this condition is hereinafter referred to as "during ejection stop"), the fluid shifts in the following manner and therefore scarcely flows out through the fluid ejection opening. During ejection stop, the fluid is continuously supplied to the fluid chamber from a fluid supply unit. However, the flow speed of the fluid decreases at the fluid ejection opening due to the larger opening area of the outlet opening end of the fluid ejection opening than the opening area of the inlet opening end of the fluid ejection opening. In this case, the flow speed of the fluid becomes a speed lower than the speed exceeding the coagulation force of the fluid. As a result, the fluid flowing out of the fluid ejection opening in the form of liquid drops is accumulated around the fluid ejection opening, and suctioned by the suction unit to be collected. Accordingly, the surgical instrument of Application Example 1 can reduce leakage of the fluid from the surgical instrument by a simplified structure.

### Application Example 2

This application example of the invention is directed to the surgical instrument of Application Example 1, wherein the fluid ejection opening has a hole through which the fluid is introduced from the fluid ejection opening toward the suction opening.

According to the surgical instrument of Application Example 2, the fluid existing inside the fluid ejection opening can be introduced through the hole toward the suction opening. Thus, the fluid remaining during pulsed flow ejection stop can be more efficiently suctioned.

### Application Example 3

This application example of the invention is directed to the surgical instrument of Application Example 1 or 2, wherein the suction opening projects toward the ejection target from the fluid ejection opening.

According to the surgical instrument of Application Example 3, the fluid remaining around the outlet opening end of the fluid ejection opening and falling in the vertical downward direction by its own weight can be received on a suction tube. Accordingly, the fluid remaining during pulsed flow ejection stop can be more efficiently suctioned.

### Application Example 4

This application example of the invention is directed to the surgical instrument of any of Application Examples 1 to 3, wherein the opening area of the fluid ejection opening increases from the inlet opening end toward the outlet opening end.

According to the surgical instrument of Application Example 4, the flow speed of the fluid gradually decreases within the fluid ejection opening. Thus, the fluid can be more easily accumulated around the fluid ejection opening.

### Application Example 5

This application example of the invention is directed to the surgical instrument of any of Application Examples 1 to 4, wherein the fluid ejection opening has a straight portion having a uniform opening area, and a conical portion whose opening area increases from one end of the straight portion toward the outlet opening end.

According to the surgical instrument of Application Example 5, the ejection direction of the pulse flow can be easily set at one direction by the guide of the straight portion of the fluid ejection opening. Thugs, the linearity of the ejection direction improves.

### Application Example 6

This application example of the invention is directed to the surgical instrument of any of Application Examples 1 to 5, which further includes a controller which selects and executes a pulsed flow ejection control for allowing the pulsation generator to repeat pressurization and depressurization of the fluid chamber, or a pulsed flow ejection stop control for stopping the pressurization and depressurization of the fluid chamber performed by the pulsation generator.

According to the surgical instrument of Application Example 6, the controller executes control during pulsed flow ejection and control during pulsed flow ejection stop.

### BRIEF DESCRIPTION OF THE DRAWINGS

The invention will be described with reference to the accompanying drawings, wherein like numbers reference like elements.

Fig. 1. illustrates the structure of a surgical instrument as a surgical instrument according to a first embodiment.

Fig. 2 illustrates a cross section of a pulsation generator and a dual tube in the first embodiment taken along the direction of fluid ejection.

Figs. 3A and 3B are a cross-sectional view and a front view of the dual tube of Figure 2.

Figs. 4A and. 4B illustrate the condition of fluid W during stop of pulsed flow ejection and during pulsed flow ejection.

Fig. 5 illustrates the condition of the fluid W suctioned into the dual tube of Figure 2.

Figs. 6A and 6B are graphs showing the change of the flow amount of fluid ejected from a fluid ejection opening during stop of pulsed flow ejection and during pulsed flow ejection.

Fig. 7 illustrates a dual tube according to a second embodiment.

Fig. 8 is a front view of a dual tube according to a third embodiment as viewed from the tip thereof.

Fig. 9 illustrates a lateral cross section of the dual tube according to the third embodiment.

Fig. 10 illustrates a lateral cross section of a dual tube according to a fourth embodiment.

Fig. 11 illustrates a lateral cross section of a dual tube according to a fifth embodiment.

Fig. 12 illustrates a lateral cross section of a dual tube according to a sixth embodiment.

Figs. 13A through 13D illustrate shapes of a fluid ejection opening according to modified examples.

### DESCRIPTION OF EXEMPLARY EMBODIMENTS

Exemplary embodiments according to the invention are hereinafter described with reference to the drawings. A surgical instrument according to the invention is of a type used in a variety of application fields such as drawing in ink or the like, cleaning of a minute object or structure, and use as a surgical knife. In the following embodiments, a surgical instrument particularly suited for incision or excision of living tissue will be discussed. Therefore, the fluid ejected in the respective embodiments is water, physiological saline, or other fluid appropriate for this purpose.

### A. First Embodiment

### A-1. Entire Structure

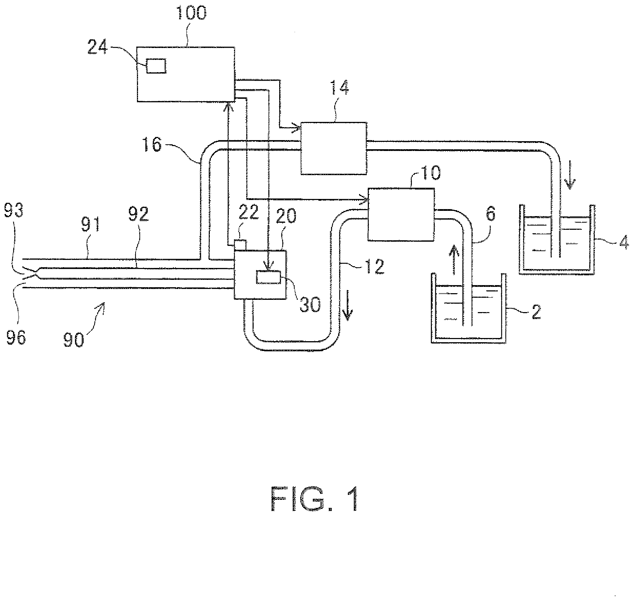

Fig. 1 illustrates the structure of a surgical instrument functioning as a surgical instrument according to a first embodiment. As illustrated in Fig. 1, a surgical instrument 1 includes a fluid container 2 (hereinafter referred to as a "fluid supply container") which stores fluid, a first pump 10 as a fluid supply unit which communicates with the fluid supply container 2, a pulsation generator 20 which converts fluid supplied from the first pump 10 into pulsed flow of fluid, and a narrow pipe-shaped dual tube 90 which connects with the pulsation generator 20. The first pump 10 and the pulsation generator 20 are connected with each other via a fluid supply tube 12.

The surgical instrument 1 further includes a second pump 14 as a fluid suction unit which suctions fluid from the dual tube 90, and a fluid container 4 (hereinafter referred to as a "fluid collection container") which stores fluid suctioned by the second pump 14. The second pump 14 is connected with the dual tube 90 via a fluid collection tube 16.

The dual tube 90 has a dual cylindrical tube structure which inserts a small-diameter cylindrical tube (inside tube) 92 into a large-diameter cylindrical tube (outside tube) 91. A fluid ejection opening 93 through which fluid is ejected is provided at the tip of the small-diameter cylindrical tube 92. A suction opening 96 through which fluid is suctioned is provided at the tip of the space between the large-diameter cylindrical tube 91 and the small-diameter cylindrical tube 92. The area inside the small-diameter cylindrical tube 92 communicates with the pulsation generator 20, while the area between the large-diameter cylindrical tube 91 and the small-diameter cylindrical tube 92 communicates with the fluid collection tube 16.

The dual tube 90 is rigid enough not to be deformed during ejection of fluid. It is preferable that the fluid supply tube 12 and the fluid collection tube 16 are flexible and bendable.

It is further preferable that the fluid ejection opening 93 is lyophilic. When the fluid ejection opening 93 is lyophilic, liquid drops remaining on the fluid ejection opening 93 do not easily fall from the fluid ejection opening 93 but can be sucked through the suction opening 96. The details of this mechanism will be described later.

The surgical instrument 1 further includes a controller 100 which controls the driving of the first pump 10, the second pump 14, and the pulsation generator 20. The pulsation generator 20 has a pulsed flow ejection switch 22 through which the on/off condition of the pulsed flow ejection is controlled. The controller 100 has a main switch 24 through which the on/off condition of the surgical instrument 1 is controlled. The pulsed flow ejection switch 22 and the main switch 24 are operated by an operator of the surgical instrument 1.

The controller 100 receives on/off commands from the main switch 24 and the pulsed flow ejection switch 22, and drives or stops the first pump 10, the second pump 14, and the pulsation generator 20 in accordance with the received commands. More specifically, the controller 100 drives the first pump 10 and the second pump 14 when the main switch 24 is turned on, and stops the first pump 10 and the second pump 14 when the main switch 24 is turned off. Also, the controller 100 drives the pulsation generator 20 when the pulsed flow ejection switch 22 is turned on under the ON condition of the main switch 24, and stops the drive of the pulsation generator 20 when the pulsed flow ejection switch 22 is turned off. Thus, the pulsed flow ejection switch 22 selects and executes the drive (pulsed flow ejection ON control) or the stop (pulsed flow ejection OFF control) of the pulsation generator 20.

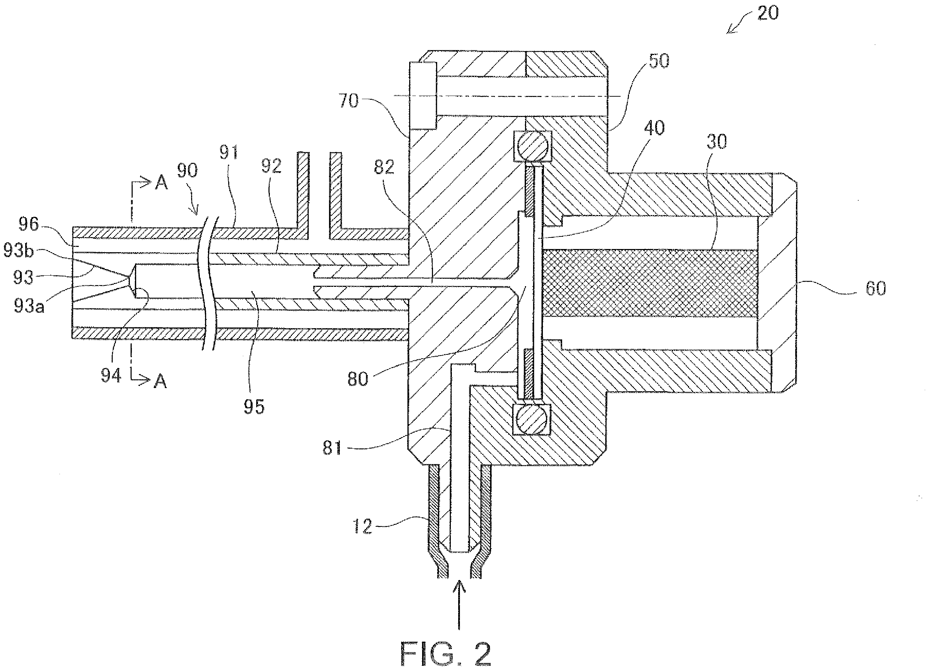

When the first pump 10 is driven, fluid stored in the fluid supply container 2 is suctioned by the first pump 10 via a connection tube 6, and supplied under a constant pressure to the pulsation generator 20 via the fluid supply tube 12. The pulsation generator 20 has a fluid chamber 80 (see Fig. 2) and a volume changing unit (described later) which changes the volume of the fluid chamber 80. When the pulsation generator 20 is driven, the fluid chamber 80 generates pulsed flow which allows flow of fluid through the small-diameter cylindrical tube 92 of the dual tube 90 and ejection of the fluid in pulses from the fluid ejection opening 93. The detailed structure of the pulsation generator 20 including the volume changing unit will be described later. During pulsed flow ejection, the second pump 14 operates as well. Thus, the fluid remaining at the end of the suction opening 96 of the dual tube 90 is suctioned by the second pump 14 via the fluid collection tube 16, and collected into the fluid collection container 4.

The term "pulsed flow" herein refers to flow of fluid which has a constant flow direction and a flow amount or a flow speed variable periodically or irregularly. The pulsed flow includes intermittent flow which repeatedly starts and stops fluid flow. However, the pulsed flow may be other types of flow as long as the flow amount or the flow speed of fluid varies periodically or irregularly.

The term "fluid ejection in pulses" herein refers to ejection of fluid whose flow amount or moving speed varies periodically or irregularly. In this specification, the fluid ejection in pulses is also expressed as "pulsed flow ejection,". The ejection in pulses includes intermittent ejection which repeats ejection and non-ejection of fluid, for example. However, the ejection in pulses may be other types of ejection as long as the flow amount or the moving speed of fluid varies periodically or irregularly.

When the driving of the pulsation generator 20 stops under the control of the controller 100 during driving of the first pump 10 and the second pump 14, the fluid continues flowing from the fluid ejection opening 93 due to by the driving of the first pump 10 even along with the stopping of the pulsed flow ejection from the fluid ejection opening 93. The fluid coming from the fluid ejection opening 93 is accumulated in the vicinity of the tip of the fluid ejection opening 93, and suctioned from the suction opening 96 through the fluid collection tube 16 toward the second pump 14 by the operation of the second pump 14. The fluid is thereby collected into the fluid collection container 4.

According to this embodiment, the controller 100 is constituted by a known microcomputer which contains a CPU, a ROM, a RAM (not shown) and others. The controller 100 is not limited to a microcomputer, but may be constituted by a combination of discrete electronic components.

Moreover, the function of the first pump 10 for supplying the sufficient flow amount of fluid to the pulsation generator 20 may be provided by such a structure which attaches a liquid transportation bag to a stand or the like and holds the bag at a higher position than the pulsation generator 20. In this case, the elimination of the first pump 10 simplifies the structure, and facilitates disinfection and other treatments. It is possible to use both the liquid transportation bag and the first pump 10 at the same time.

During surgery with the aid of the surgical instrument 1 thus constructed, the dual tube 90 is pointed to target living tissue, whereafter ejection of fluid in pulses is executed from the fluid ejection opening 93 to incise or excise the living tissue.

### A-2. Structure of Pulsation Generator 20 and Dual Tube 90

Fig. 2 is a cross-sectional view showing a cross section of the pulsation generator 20 and the dual tube 90 according to this embodiment, taken along the fluid ejection direction. Fig. 2 is a schematic illustration whose components and parts have different horizontal and vertical scaling as compared to actual for convenience of explanation. The pulsation generator 20 includes an inlet channel 81 through which fluid supplied from the first pump 10 (Fig. 1) via the fluid supply tube 12 flows toward the fluid chamber 80, a piezoelectric device 30 and a diaphragm 40 as the volume changing unit, and an outlet channel 82 communicating with the fluid chamber 80.

The diaphragm 40 as a disk-shaped metal thin plate tightly contacts a case 50 and a case 70. According to this embodiment, the piezoelectric device 30 is constituted by a laminated-type piezoelectric device, one end of which is fixed to the diaphragm 40, while the other end of which is fixed to a bottom plate 60.

The fluid chamber 80 is a space produced by the diaphragm 40 and a recess formed in the surface of the case 70 on the side facing to the diaphragm 40. The outlet channel 82 is opened substantially at the center of the fluid chamber 80.

The case 70 and the case 50 are joined to each other via the opposed surfaces of the cases 70 and 50 to be combined into one body. The dual tube 90 engages with the case 70 in such a condition that a connection channel 95 of the dual tube 90 communicates with the outlet channel 82. While the cross-sectional channel shape of the outlet channel 82 is different from the shape of the connection channel 95 in this embodiment, the outlet channel 82 and the connection channel 95 may have the same cross-sectional channel shape. That is, the outlet channel 82 and the connection channel 95 may be provided as one continuous outlet channel.

As explained above, the dual tube 90 has a dual cylindrical tube structure which inserts the small-diameter cylindrical tube 92 into the large-diameter cylindrical tube 91, and includes the connection channel 95 corresponding to the inside of the small-diameter cylindrical tube 92. The center axis of the large-diameter cylindrical tube 91 aligns with the center axis of the small-diameter cylindrical tube 92 (that is, the dual tube 90 has a concentric dual cylindrical tube structure).

The fluid ejection opening 93 is provided at the tip of the small-diameter cylindrical tube 92, and connected with one end of the connection channel 95. The other end of the connection channel 95 is connected with the outlet channel 82.

The fluid ejection opening 93 has a hole shape which has an inlet opening end 93a on the side near the outlet channel 82, and an outlet opening end 93b on the side near the outside. The fluid ejection opening 93 is disposed such that the center axis direction of the hole agrees with the center axis direction of the connection channel 95 (i.e., the center axis direction of the small-diameter cylindrical tube 92). The fluid ejection opening 93 has a shape expanded in the direction from the inlet opening end 93a toward the outlet opening end 93b, and the opening area of the outlet opening end 93b is larger than the opening area of the inlet opening end 93a.

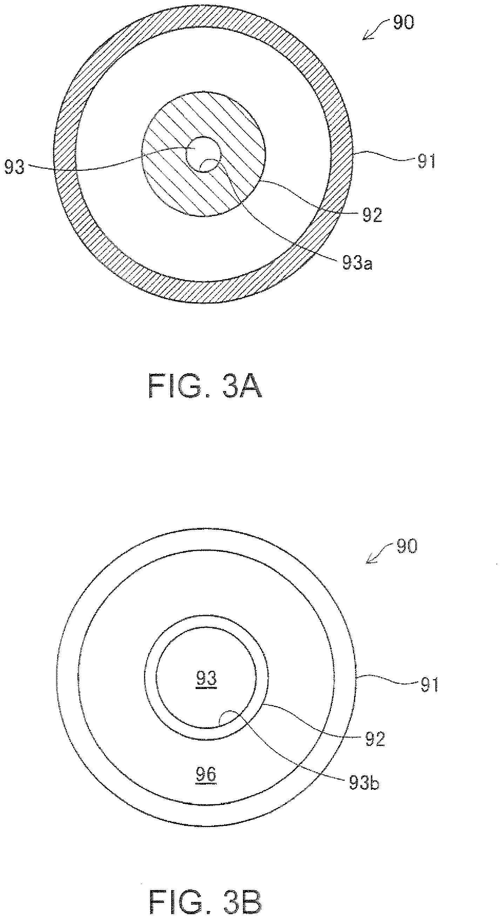

Figs. 3A and 3B illustrate the cross section and the front of the dual tube 90, respectively. Fig. 3A is a cross-sectional view taken along line A-A in Fig. 2 as viewed in the direction of arrows, while Fig. 3B is a front view as viewed from the tip of the dual tube 90. As can be seen from Figs. 3A and 3B, the opening area of the outlet opening end 93b is larger than the opening area of the inlet opening end 93a. According to the first embodiment, the diameter of the inlet opening end 93a is in the range of from 0.1 mm to 0.3 mm, while the diameter of the outlet opening end 93b is in the range of from 1 mm to 3 mm. The length of the fluid ejection opening 93 in the center axis direction is in the range of from 1 mm to 5 mm.

As illustrated in Fig. 2, a connection surface 94 between the inlet opening end 93a and the one end of the connection channel 95 extends not in the vertical direction with respect to the center axis direction of the connection channel 95, but in such a direction that the angle formed by the connection surface 94 and the inner wall surface of the connection channel 95 becomes an obtuse angle. According to this structure, accumulation of bubbles at the corners is prevented. The corners formed by the connection surface 94 and the inner wall surface of the connection channel 95 may be rounded. In this case, accumulation of bubbles at the corners can be similarly prevented. By elimination of bubbles remaining at the corners, attenuation of pressure of the pulsed flow transmitted through the inside of the connection channel 95 caused by the existence of bubbles can be avoided.

When a driving signal (which alternately repeats peaks and troughs) is inputted to the piezoelectric device 30, the piezoelectric device 30 repeats expansion and contraction in accordance with the driving waveform of the driving signal. When the piezoelectric device 30 expands, the volume of the fluid chamber 80 decreases. When the piezoelectric device 30 contracts, the volume of the fluid chamber 80 increases. These repeated actions of the fluid chamber 80 generate pulsed flow. By generation of the pulsed flow, fluid continuously comes out through the outlet channel 82 at high speed in the form of pulsed liquid drops, and moves along the connection channel 95 to be ejected through the fluid ejection opening 93. That is, fluid flows out as pulsed flow ejection. According to the first embodiment, the frequency of the expansion and contraction of the piezoelectric element 30 is set in the range of from 1 Hz to 10 kHz.

### A-3. Condition of Fluid after Ejection from Fluid Ejection Opening 93, and Advantages of First Embodiment

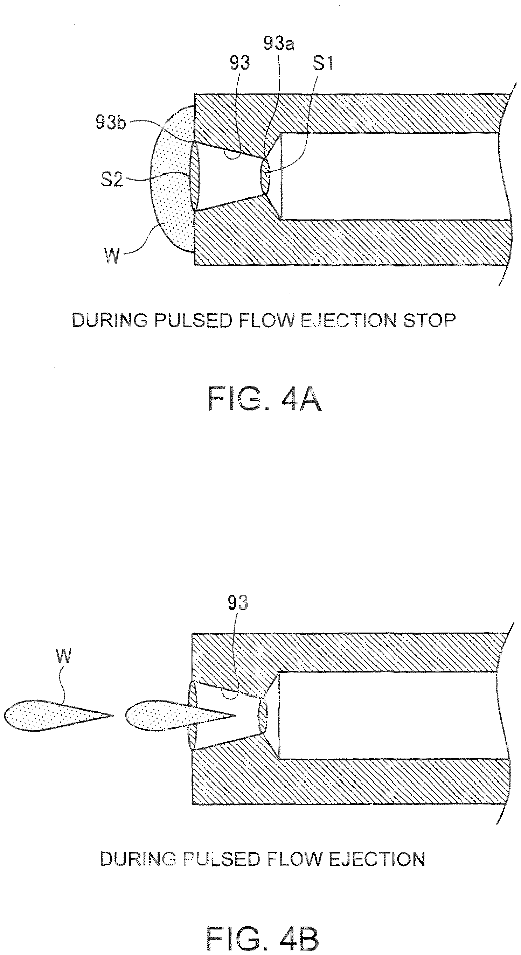

Figs. 4A and 4B illustrate the condition of fluid during stop of pulsed flow ejection and during pulsed flow ejection. Fig. 4A corresponds to the condition during stopping of the pulsed flow ejection, while Fig. 4B corresponds to the condition during pulsed flow ejection.

When a constant flow amount of fluid is supplied by the first pump 10 during stop of pulsed flow ejection, the flow speed of the fluid gradually decreases as the fluid at the fluid ejection opening 93 approaches the outlet opening end 93b under the law of continuity of fluid which holds by the shape of the fluid ejection opening 93 gradually expanding in a cone shape from the inlet opening end 93a to the outlet opening end 93b as illustrated in Fig. 4A. In this case, the fluid coagulates and does not flow out from the fluid ejection opening 93 due to the coagulation force of the fluid generated by the intermolecular force, i.e., the surface tension when the flow speed does not exceed the coagulation force. The word "flow out" herein refers to the action of the fluid going out and away from the dual tube 90 after ejection of the fluid. Thus, the fluid having a low ejection speed accumulates in the vicinity of the outlet opening end 93b, or the liquid drops of the fluid remaining thereat adhere to the inner wall surface of the fluid ejection opening 93. Moreover, the fluid remaining in the vicinity of the outlet opening end 93b and the fluid adhering to the inner wall surface of the fluid ejection opening 93 become cores collecting further non-ejected fluid. As a result, more fluid accumulates and produces fluid W remaining in the vicinity of the outlet opening end 93b of the fluid ejection opening 93 as illustrated in Fig. 4A.

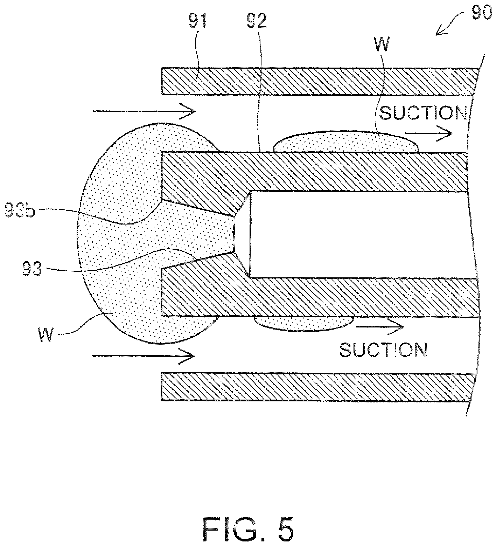

During stop of pulsed flow ejection, the second pump 14 continues operation. Thus, the fluid W remaining in the vicinity of the outlet opening end 93b of the fluid ejection opening 93 is sucked from the suction opening 96 through the space between the large-diameter cylindrical tube 91 and the small-diameter cylindrical tube 92 as illustrated in Fig. 5.

During pulsed flow ejection, the flow amount of fluid supplied from the fluid chamber 80 to the fluid ejection opening 93 rapidly increases at the rise of the driving waveform. In this case, fluid does not flow in contact with the inner wall surface of the fluid ejection opening 93 but separates therefrom as illustrated in Fig. 4B. During the subsequent fall of the driving waveform, ejection of fluid through the fluid ejection opening 93 stops until the same amount of fluid as that of the ejected fluid fills the fluid ejection opening 93. As a consequence, continuous ejection of the fluid W from the fluid ejection opening 93 (in the form of pulsed liquid drops) can be achieved as illustrated in the figure.

According to this structure, the fluid ejection opening 93 has a conical shape which gradually expands from the inlet opening end 93a toward the outlet opening end 93b. In this case, the opening area of the outlet opening end 93b can be enlarged while maintaining the small opening area of the inlet opening end 93a which has great correlation with the flow out of fluid during pulsed flow ejection. Thus, fluid ejection in pulses can be achieved without lowering the effect of pulsed ejection.

Accordingly, the surgical instrument 1 in the first embodiment offers advantages of (i) the fluid W can be kept remaining at the fluid ejection opening 93 without separation therefrom during the stopping of the pulsed flow ejection, and (ii) fluid ejection in pulses can be achieved without lowering the effect of pulsed ejection during pulsed flow ejection, both advantages of which can be provided by a simple structure.

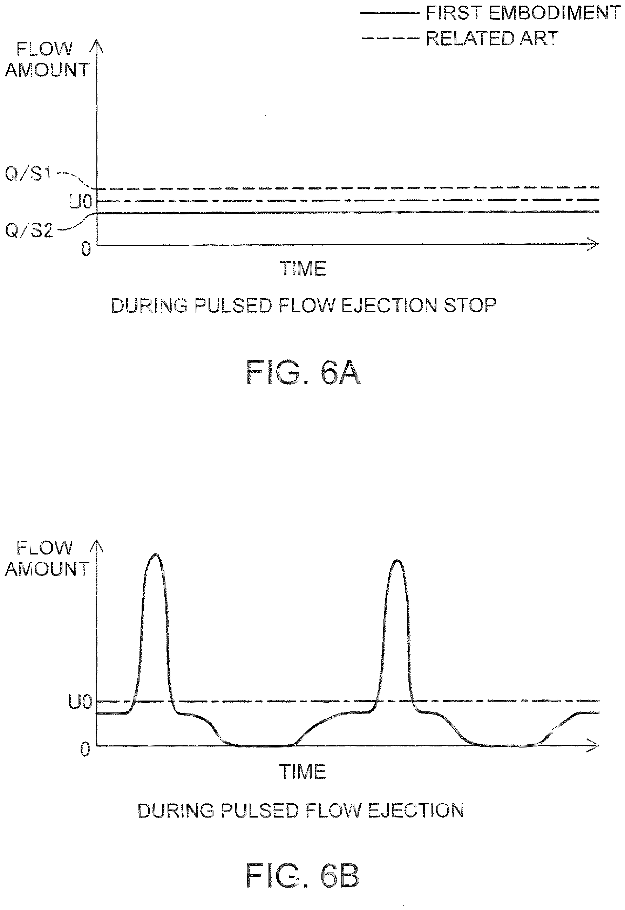

The change of the flow amount of fluid ejected from the fluid ejection opening 93 during pulsed flow ejection and during stop of pulsed flow ejection is now explained with reference to graphs shown in Figs. 6A and 6B. Fig. 6A shows the change of the flow amount during stopping of the pulsed flow ejection with an elapse of time, while Fig. 6B shows the change of the flow amount during pulsed flow ejection with an elapse of time. The vertical axis in each of the graphs in Figs. 6A and 6B indicates the flow amount per unit area at the fluid ejection opening 93, while the horizontal axis indicates time.

Assuming that the opening area of the outlet opening end 93b of the fluid ejection opening 93 is S2, and that the supply amount from the first pump 10 is Q, the flow amount during stop of pulsed flow ejection becomes a constant value calculated from Q/S2 as indicated by a solid line in Fig. 6A. A value U0 in the graph corresponds to a threshold of the flow speed at which fluid can flow out from the fluid ejection opening 93. As can be seen from the graph, the value Q/S2 is smaller than the threshold U0. Thus, the fluid does not flow out from the fluid ejection opening 93 even while supply of the fluid flow amount from the first pump 10 continues. According to the first embodiment, the supply amount Q from the first pump 10 and the opening area of the outlet opening end 93b of the fluid ejection opening 93 are determined such that the relation U0 > Q/S2 holds.

A dotted line in Fig. 6A indicates the flow amount in a related art (the flow amount ejected from a fluid ejection opening per unit area during the stopping of the pulsed flow ejection). The related art compared herein is constructed such that the fluid ejection opening has a uniform inside diameter throughout the area thereof. It is assumed that the inside diameter of the fluid ejection opening in the related art is equivalent to that of an inlet area S1 of the inlet opening end 93a in the first embodiment. The flow amount of fluid in the related art becomes a constant value calculated from Q/S1. As can be seen from the figure, the value Q/S1 is larger than the threshold U0 of the flow speed at which fluid can flow out from the fluid ejection opening. In the case of the related art, therefore, fluid is ejected from the fluid ejection opening even at the stop of pulsed flow ejection.

During pulsed flow ejection, the flow amount immediately increases as shown in Fig. 6B, in which condition fluid flows out from the fluid ejection opening 93. The peak of the flow amount during this period is far larger than the threshold U0. Since the flow amount (which is larger than the flow amount Q supplied by the first pump 10) is instantly ejected (together with the fluid pulled and ejected by the inertial force of the fluid), all the fluid remaining on the fluid ejection opening 93 is ejected. As a result, the flow amount of fluid from the fluid ejection opening 93 becomes zero until the same amount of fluid as that of the ejected fluid fills the fluid ejection opening 93. Accordingly, ejection of the fluid W can be achieved in the form of pulsed flow.

As apparent from the description with reference to Figs. 6A and 6B, a wide opening of the fluid ejection opening is required so as to prevent flow out of fluid from the fluid ejection opening during stopping of the pulsed flow ejection. In this embodiment, the fluid ejection opening 93 has a conical shape which gradually expands from the inlet opening end 93a toward the outlet opening end 93b. According to this structure, flow out of fluid from the fluid ejection opening 93 during stop of pulsed flow ejection is prevented by the increased opening area of the outlet opening end 93b having correlation with leakage of fluid, while high-speed ejection of fluid in pulses can be achieved during pulsed flow ejection by the decreased opening area of the inlet opening end 93a having correlation with ejection of fluid without requiring rise of the expanding and contracting capacity of the piezoelectric device 30 as the volume changing unit.

### B. Second Embodiment

Second through sixth embodiments and modified examples 1 through 5 are hereinafter described. In the following description, the parts different from the corresponding parts in the first embodiment are only discussed. The structures similar to the corresponding structures in the first embodiment have been given similar reference numbers, and the same explanation of those is not repeated.

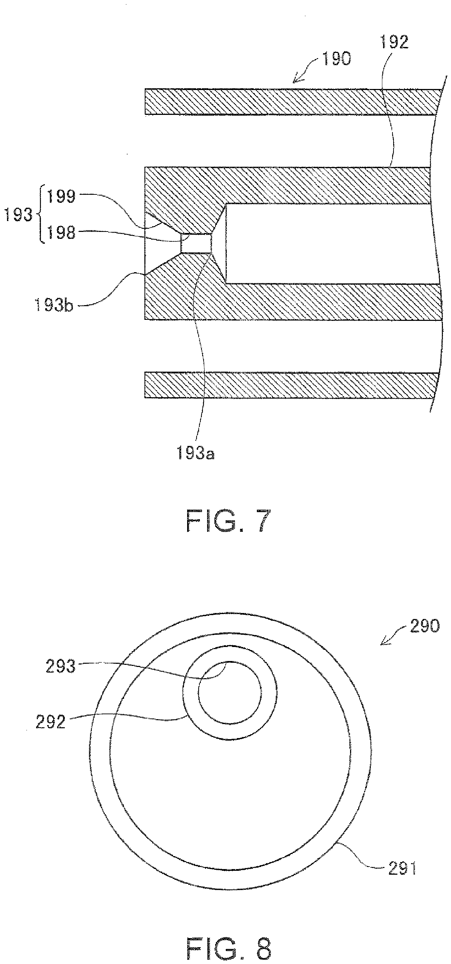

Fig. 7 illustrates a dual tube 190 included in a surgical instrument according to the second embodiment. The surgical instrument in the second embodiment has structures similar to the corresponding structures of the surgical instrument 1 in the first embodiment except for the shape of a fluid ejection opening 193 included in the surgical instrument in the second embodiment.

As illustrated in the figure, the dual tube 190 has a concentric dual cylindrical tube structure similarly to the first embodiment. The fluid ejection opening 193 is provided at the tip of a small-diameter cylindrical tube 192. The fluid ejection opening 193 has a straight portion 198 having a uniform inside diameter, and a conical portion 199 (conical portion) which has a conical shape gradually expanding from one end of the straight portion 198 toward an outlet opening end 193b of the fluid ejection opening 193. The center axis direction of the straight portion 198 aligns with the center axis direction of the conical portion 199. The center axis directions of both the portions 198 and 199 align with the center axis direction of a connection channel 195 (i.e., the center axis direction of the small-diameter cylindrical tube 192). The end of the straight portion 198 on the side opposite to the conical portion 199 corresponds to an inlet opening end 193a of the fluid ejection opening 193.

According to the surgical instrument in the second embodiment thus constructed, fluid stops without flow out from the fluid ejection opening 193 under the condition of the first pump 10 not completely stopping, similar to the first embodiment. Moreover, according to the surgical instrument in the second embodiment, the ejection direction of the pulsed flow can be easily set at one direction by the guide of the straight portion 198 of the fluid ejection opening 193. Thus, the linearity of the ejection direction improves.

### C. Third Embodiment

Fig. 8 is a front view of a surgical instrument according to a third embodiment as viewed from the tip of a dual tube 290. The surgical instrument in the third embodiment has structures similar to the corresponding structures of the surgical instrument 1 in the first embodiment except for the shape of the dual tube 290 included in the surgical instrument in the third embodiment. While the dual tube 90 in the first embodiment has a concentric dual cylindrical tube structure, the dual tube 290 in the third embodiment has an eccentric dual cylindrical tube structure as illustrated in the figure. More specifically, according to the dual tube 290, the center axis of an inside cylindrical tube 292 is shifted from the center axis of an outside cylindrical tube 291. A fluid ejection opening 293 configured similarly to the corresponding part in the first embodiment is provided at the tip of the inside cylindrical tube 292 of the dual tube 290 thus constructed.

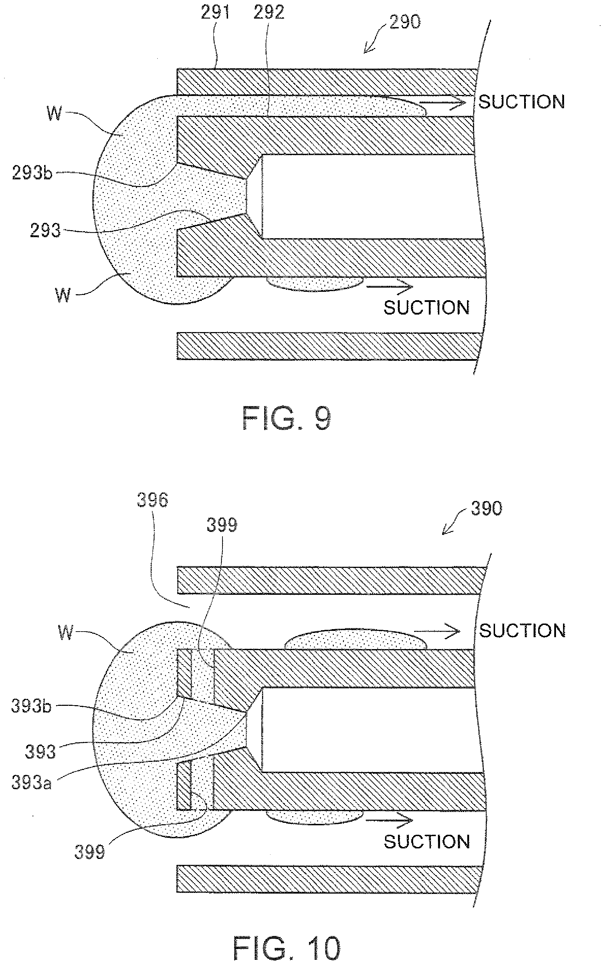

Fig. 9 illustrates a lateral cross section of the dual tube 290 in the third embodiment. According to the surgical instrument in the third embodiment, the fluid W accumulates in the vicinity of an outlet opening end 293b of the fluid ejection opening 293 during stopping of the fluid ejection similar to the surgical instrument 1 in the first embodiment. Since the inside cylindrical tube 292 is eccentrically disposed with respect to the outside cylindrical tube 291, a capillary phenomenon is produced in a narrower space between the outside cylindrical tube 291 and the inside cylindrical tube 292. In this case, fluid more easily flows toward the narrower space between the outside cylindrical tube 291 and the inside cylindrical tube 292 due to the generated capillary phenomenon, whereby the remaining fluid W can be more easily suctioned.

### D. Fourth Embodiment

Fig. 10 illustrates a lateral cross section of a dual tube 390 of a surgical instrument according to a fourth embodiment. The surgical instrument in the fourth embodiment has structures similar to the corresponding structures of the surgical instrument 1 in the first embodiment except for the shapes of a fluid ejection opening 393 and its surroundings included in the surgical instrument in the fourth embodiment.

As illustrated in the figure, the dual tube 390 has a concentric dual cylindrical tube structure similarly to the first embodiment. The fluid ejection opening 393 is provided at the tip of an inside cylindrical tube 392. The fluid ejection opening 393 has a conical shape which gradually expands from an inlet opening 393a toward an outlet opening end 393b similarly to the first embodiment. Thus, the opening area of the outlet opening end 393b is larger than the opening area of the inlet opening end 393a. According to the fourth embodiment, a plurality of through holes 399 (holes) are formed in the middle of the inner wall surface of the fluid ejection opening 393 and extended to the outer wall surface of the inside cylindrical tube 392. The through holes 399 are provided in directions perpendicular to the center axis of the inside cylindrical tube 392. According to the fourth embodiment, the four through holes 399 (only two of which are shown in the figure) are extended radially in four directions along one plane perpendicular to the center axis of the inside cylindrical tube 392.

According to the fourth embodiment having this structure, fluid existing inside the fluid ejection opening 393 can be introduced toward a suction opening 396 through the through holes 399. Thus, the fluid W remaining during stop of pulsed flow ejection can be more easily suctioned.

### E. Fifth Embodiment

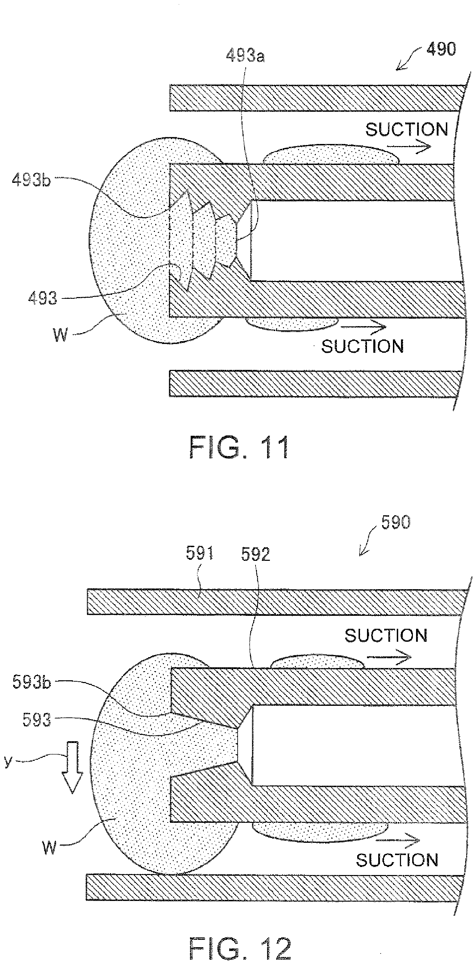

Fig. 11 illustrates a lateral cross section of a dual tube 490 of a surgical instrument according to a fifth embodiment. The surgical instrument in the fifth embodiment has structures similar to the corresponding structures of the surgical instrument 1 in the first embodiment except for the shape of a fluid ejection opening 493 included in the surgical instrument in the fifth embodiment.

As illustrated in the figure, the fluid ejection opening 493 provided on a dual tube 490 is different from the corresponding component in the first embodiment in that concave and convex structured are provided on the inner wall surface of the fluid ejection opening 493 in the area from an inlet opening end 493a to an outlet opening end 493b. According to the fifth embodiment thus constructed, fluid can be accumulated on the concave structures formed on the inner wall surface in the area from the inlet opening end 493a to the outlet opening end 493b. Thus, fluid can be easily stopped at the fluid ejection opening 493.

### F. Sixth Embodiment

Fig. 12 illustrates a lateral cross section of a dual tube 590 of a surgical instrument according to a sixth embodiment. The surgical instrument in the sixth embodiment has structures similar to the corresponding structures of the surgical instrument 1 in the first embodiment except for the shape of a dual tube 590 included in the surgical instrument in the sixth embodiment.

As illustrated in the figure, the dual tube 590 has a concentric dual cylindrical tube structure similar to the first embodiment, and has a fluid ejection opening 593 disposed at the tip of an inside cylindrical tube 592. The fluid ejection opening 593 is identical to the corresponding component in the first embodiment. The sixth embodiment is different from the first embodiment in that the tip of an outside cylindrical tube 591 is projected from the tip of the inside cylindrical tube 592 in the fluid ejection direction. According to the sixth embodiment thus constructed, the fluid W remaining in the vicinity of an outlet opening end 593b of the fluid ejection opening 593 and falling by its own weight in a vertical downward direction y can be received on the outside cylindrical tube 591. This fluid W is suctioned through the space between the outside cylindrical tube 591 and the inside cylindrical tube 592. Therefore, leakage of fluid during stop of pulsed flow ejection can be securely prevented according to the sixth embodiment.

### G. Modified Examples

The invention is not limited to the respective first through sixth embodiments and other modified examples but may be practiced otherwise without departing from the scope of the invention. For example, the following changes may be made.

### Modified Example 1

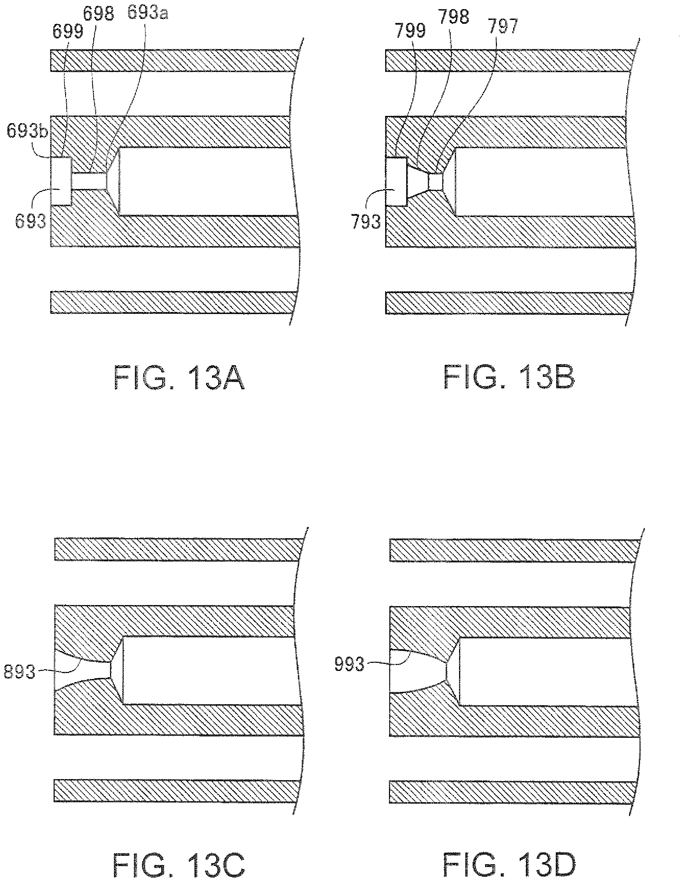

According to the first embodiment, the fluid ejection opening 93 has a conical shape which gradually expands from the inlet opening end 93a to the outlet opening end 93b. However, the fluid ejection opening 93 may have other shapes as long as the opening area of the outlet opening end 93b is larger than the opening area of the inlet opening end 93a. Figs. 13A through 13D show examples of other shapes of the fluid ejection opening.

As illustrated in Fig. 13A, a fluid ejection opening 693 may have two straight portions 698 and 699 having different inside diameters and connected with each other. According to this structure, the second straight portion 699 disposed near an outlet opening end 693b has a larger inside diameter than that of the first straight portion 698 disposed near an inlet opening end 693a..

As illustrated in Fig. 13B, a fluid ejection opening 793 may have a straight portion (first straight portion) 797 and a conical portion 798 similar to the corresponding portions in the second embodiment, and further a second straight portion 799 on the fluid ejection side of the conical portion 798. According to this structure, the inside diameter of the second straight portion 799 is larger than that of the first straight portion 797.

Moreover, the inclination of the slant line of the conical portion of a fluid ejection opening 893 with respect to the center axis is not required to be constant but may be varied in such a manner that the rate of inclination change increases toward the fluid ejection side as illustrated in Fig. 13C. Alternatively, such a fluid ejection opening 993 whose rate of inclination change decreases toward the fluid ejection side may be provided as illustrated in Fig. 13D.

### Modified Example 2

According to the respective embodiments and modified examples, pulsed flow is generated in accordance with the press of the diaphragm 40 by the piezoelectric device 30. However, other structures may be adopted as long as pulsed flow can be generated. For example, the volume of the fluid chamber 80 may be reduced in accordance with the movement of a piston (plunger) driven by a piezoelectric device to produce pulsed flow. Moreover, the volume of the fluid chamber 80 is not required to be varied as long as the pressure of the fluid supplied to the fluid chamber 80 can be increased or decreased. For example, the fluid within the fluid chamber 80 may be pressurized by bubbles produced by laser induction or heater electrodes to generate pulsed flow.

### Modified Example 3

According to the respective embodiments and modified examples, the dual tube 90 has a dual cylindrical tube structure for suctioning fluid remaining around the fluid ejection opening 93. However, this fluid may be suctioned through a nozzle provided with a suction opening disposed in the vicinity of the fluid ejection opening 93. As such, any suctioning structure may be adopted as long as the suction opening is provided in the vicinity of the fluid ejection opening as a suction opening through which fluid remaining around the fluid ejection opening is suctioned. In other words, the suction opening may be located at any position close enough to suction the fluid remaining around the fluid ejection opening.

### Modified Example 4

While the cylindrical tubes 91 and 92 constituting the dual tube 90 have cylindrical shapes according to the respective embodiments and modified examples, these tubes 91 and 92 may have polygonal columnar shapes.

### Modified Example 5

According to the respective embodiments and modified examples, the supply amount of the first pump 10 as the fluid supply unit is equalized for both periods of pulsed flow ejection and stop of pulsed flow ejection. However, the supply amount of fluid from the first pump 10 during stopping of the pulsed flow ejection may be set smaller than the supply amount of fluid from the first pump 10 during pulsed flow ejection. According to this structure, leakage of fluid during stopping of the pulsed flow ejection can be further decreased.

### Modified Example 6

According to the respective embodiments and modified example, hydrophilic treatment may be applied to the inner wall surface of the fluid ejection opening from the inlet opening end to the outlet opening end. The hydrophilic treatment avoids easy separation of fluid remaining on the inner wall surface from this wall surface. Accordingly, leakage of fluid during stop of pulsed flow ejection can be further decreased.

The constituent elements included in the respective embodiments and modified examples other than the elements claimed in the appended independent claims are only supplemental elements, and therefore can be eliminated when appropriate.

## Claims

1. A surgical instrument comprising:
a fluid chamber to which fluid is supplied;
a pulsation generator which produces pulsed flow by pressurizing the fluid supplied to the fluid chamber;
an outlet channel which communicates with the fluid chamber through which the pulsed flow is transmitted;
a fluid ejection opening which communicates with the outlet channel through which pulse flow is ejected toward an ejection target;
a suction opening provided in the vicinity of the fluid ejection opening; and
a fluid suction unit which suctions the fluid through the suction opening,
wherein the opening area at an outlet opening end of the fluid ejection opening on the side near the ejection target is larger than the opening area at an inlet opening end of the fluid ejection opening on the side near the outlet channel.

2. The surgical instrument according to claim 1, wherein the fluid ejection opening has a hole through which the fluid is introduced from the fluid ejection opening toward the suction opening.

3. The surgical instrument according to claim 1 or 2, wherein the suction opening projects toward the ejection target from the fluid ejection opening.

4. The surgical instrument according to claim 1, 2 or 3 wherein the opening area of the fluid ejection opening increases from the inlet opening end toward the outlet opening end.

5. The surgical instrument according to any one of the preceding claims, wherein the fluid ejection opening has a straight portion having a uniform opening area, and a conical portion whose opening area increases from one end of the straight portion toward the outlet opening end.

6. The surgical instrument according to any one of the preceding claims, wherein the center axis at the tip of the fluid ejection opening is shifted from the center axis at the tip of the suction opening.

7. The surgical instrument according to any one of the preceding claims, further comprising a controller which selects and executes a pulsed flow ejection control for allowing the pulsation generator to repeat pressurization and depressurization of the fluid chamber, or a pulsed flow ejection stop control for stopping the pressurization and depressurization of the fluid chamber performed by the pulsation generator.
